# EUROPEAN PATENT SPECIFICATION

(11) **EP 3 383 402 B1**
(45) Date of publication and mention of the grant of the patent: **24.03.2021**
(21) Application number: 16808570.2
(22) Date of filing: 30.11.2016
(51) Int. Cl.: A61K 31/635, A61K 9/20, A61P 31/18

(54) **PHARMACEUTICAL COMPOSITION COMPRISING DARUNAVIR AND METHOD FOR THE PREPARATION THEREOF**
PHARMAZEUTISCHE ZUSAMMENSETZUNG MIT DARUNAVIR UND VERFAHREN ZUR HERSTELLUNG DAVON
COMPOSITION PHARMACEUTIQUE COMPRENANT DU DARUNAVIR ET SON PROCÉDÉ DE PRÉPARATION

(30) Priority: 02.12.2015 GR 20150100526
(43) Date of publication of application: 10.10.2018
(73) Proprietor: Pharmathen S.A., 15351 Pallini Attikis (GR)
(72) Inventor: KARAVAS, Evangelos, 153 51 Pallini Attikis (GR); KOUTRIS, Efthymios, 153 51 Pallini Attikis (GR); SAMARA, Vasiliki, 153 51 Pallini Attikis (GR); KOUTRI, Ioanna, 153 51 Pallini Attikis (GR); KALASKANI, Anastasia, 153 51 Pallini Attikis (GR); KIZIRIDI, Christina, 153 51 Pallini Attikis (GR); ABATZIS, Morfis, 153 51 Pallini Attikis (GR)
(86) International application number: PCT/EP2016/025158
(87) International publication number: WO 2017/092874

(56) References cited:
- WO-A2-2013/108105

## Description

The present invention relates to oral dosage forms for the immediate release of Darunavir propylene glycolate and a method for the preparation thereof.

### BACKROUND OF THE INVENTION

Human immunodeficiency virus (HIV) is a lentivirus that has two major species, HIV-1 which causes the majority of the epidemic, and HIV-2, a close relative whose distribution is concentrated in western Africa. In 1985, HIV was identified as the causative agent of acquired immune deficiency syndrome (AIDS) and its complete genome was immediately available. This knowledge paved the way for the development of selective inhibitors.

After the discovery of HIV protease it only took 10 years for its first inhibitor to reach the market. The first reports of highly selective antagonists against the HIV protease were revealed in 1987. Phase I trials of saquinavir began in 1989 and it was the first HIV protease inhibitor to be approved for prescription use in 1995. Protease inhibitors (PIs) are a class of antiviral drugs that are widely used to treat HIV/AIDS and hepatitis caused by hepatitis C virus. Protease inhibitors prevent viral replication by selectively binding to viral proteases and blocking proteolytic cleavage of protein precursors that are necessary for the production of infectious viral particles.

A second generation of PIs was introduced later to deal with severe side effects, drug toxicity and the susceptibility of the 1^{st} generation PIs to create drug resistance mutations. Darunavir is a 2^{nd} generation PI that received much attention at the time if its release as it represents an important treatment option for patients with drug resistance HIV.

Darunavir and especially the propylene glycolate solvate has the chemical name [(1S,2R)-3-[[(4-aminophenyl) sulfonyl] (2- methyl- propyl) amino]-2-hydroxy-1-(phenyl methyl)- propyl] carbamic acid (3R,3aS,6aR)-hexahydro-furo[2,3-b]furan-3-ylester.1,2-propanediol solvate. The molecular formula is C₂₇H₃₇N₃O₇S.C₃H₈O₂ corresponding to a molecular weight of 623.73. It is a white to off-white slightly hygroscopic powder that is sparingly soluble in methanol and practically insoluble in water.

WO-A-2009/013356 discloses a tablet formulation comprising a core containing 0.1 to 1.5% by weight (w/w) of colloidal silicon dioxide, 0.4 to 0.9% by weight (w/w) of a lubricant, Darunavir, a disintegrant and a spray-dried mixture of microcrystalline cellulose and colloidal silicon dioxide as a filler.

WO-A-2013/004816 discloses a Darunavir granulate composition consisting of the active pharmaceutical ingredient, Hypromellose and any residual water from the granulation.

WO-A-2013/108105 discloses a tablet formulation comprising Darunavir propylene glycolate, microcrystalline cellulose and Crospovidone.

Although each of the patents above represents an attempt to provide tablet dosage forms of Darunavir, there still exists a need for an alternative composition.

### SUMMARY OF THE INVENTION

It is, therefore, an object of the present invention to provide a stable immediate release oral solid dosage formulation comprising Darunavir propylene glycolate, as the active ingredient.

It is another object of the present invention to provide a composition for oral administration comprising Darunavir propylene glycolate, which is bioavailable, with sufficient self-life and good pharmacotechnical properties.

Further object of the present invention is to provide a solid dosage form for oral administration containing Darunavir propylene glycolate, which can be formulated into dosage forms of different strengths by proportionally adjusting the amounts of the pharmaceutically acceptable excipients, as well as the active compound. The dosage forms of the present invention are characterized by pharmacotechnical linearity, without having any effect on the dissolution profile and bioavailability of said drug.

A further approach of the present invention is to provide an immediate release dosage form containing Darunavir propylene glycolate which is manufactured through a fast, simple and cost-effective process.

In accordance with the above objects of the present invention, an oral immediate release tablet of Darunavir propylene glycolate is provided comprising a co-spray dried mixture of lactose and microcrystalline cellulose and additionally povidone as a binder.

According to another embodiment of the present invention, a process for the preparation of an immediate release pharmaceutical composition of Darunavir propylene glycolate for oral administration is provided comprising the following steps:
- Mixing of Darunavir propylene glycolate with a co-spray dried mixture of lactose and microcrystalline cellulose along with a disintegrant and a glidant;
- Kneading with an aqueous solution of a binder;
- Drying of the granules;
- Lubrication of the granules;
- Compression of granules into tablets;
- Applying an optional film coating on the tablets.

Other objects and advantages of the present invention will become apparent to those skilled in the art in view of the following detailed description.

### DETAILED DESCRIPTION OF THE INVENTION

For the purposes of the present invention, a pharmaceutical composition comprising an active ingredient is considered to be "stable" if said ingredient degrades less or more slowly than it does on its own and/or in known pharmaceutical compositions. As already mentioned the main object of the present invention is to provide an immediate release composition of Darunavir propylene glycolate that is simple to manufacture, bioavailable, cost effective, stable and possesses good pharmacotechnical properties.

Darunavir exists in several pseudopolymorphic forms as well as the amorphous form. The marketed drug contains Darunavir in its monoethanolate form which interconverts either to amorphous or the hydrate form upon exposure to heat and low and high humidity respectively.

In the present invention the Darunavir propylene glycolate was chosen as the pharmaceutically active ingredient and surprisingly it was found that a co-spray dried mixture of lactose and microcrystalline cellulose provides both chemical and physical stability of the finished dosage form. In addition, the use of Povidone as a binder in an amount of 1% to 5% by weight improves the compaction properties as well as the dissolution properties of the tablets of the present invention.

Preferably, the co-spray dried mixture consists of 75% by weight of lactose and 25% of microcrystalline cellulose. By treating those two excipients with this technic forms a monoparticulate system having two compaction mechanisms, brittle fracture and plastic deformation. This leads to an enhanced compaction and superior flowability of the granule in comparison to mixing the two excipients individually.

Preferably, the amount of co-spray dried mixture of lactose and microcrystalline cellulose in the composition of the present invention is between 10% and 20% by weight of the total weight the composition.

Preferably, the immediate release tablets of Darunavir propylene glycolate additionally comprise Crospovidone. This is a superdisintegrant the action of which relies on a combination of swelling and wicking of liquid into the tablet to generate rapid disintegration. Crospovidone particles are granular and porous compared to other superdisintegrant and in combination with their small size they provide high surface area. The high surface area combined with unique chemistry results in high interfacial activity that enhances the dissolution of poorly soluble drugs in a way that is not possible with other disintegrant technologies.

Preferably, the amount of Crospovidone in the composition of the present invention is between 5% and 15% by weight of the total weight the composition.

Moreover, the pharmaceutical compositions of the present invention may also contain one or more additional formulation excipient such as diluents, disintegrants, binders, lubricants and glidants, provided that they are compatible with the active ingredient of the composition, so that it does not interfere with it in the composition and in order to increase the stability of the drug and the self-life of the pharmaceutical product.

Diluents may be, for example, , dextrates, dextrose, fructose, mannitol, sorbitol, starch, pregelatinized starch, sucrose, xylitol, maltose, maltodextrin, maltitol.

Disintegrants may be selected from alginic acid, carbon dioxide, carboxymethylcellulose calcium, carboxymethylcellulose sodium, croscarmelose sodium, guar gum, methylcellulose, polacrilin potassium, poloxamer, sodium alginate.

Binders may be, for example, alginic acid, carbomer, ethyl cellulose, gelatin, liquid glucose, guar gum, hydroxyethyl cellulose, povidone, methylcellulose, polydextrose, polyethylene oxide.

Also, at least a lubricant is incorporated into the formulation to prevent the powder from adhering to tablet punches during the compression procedure. Lubricants may be, for example, talc, magnesium stearate, calcium stearate, glyceryl behenate, hydrogenated castor oil, stearic acid, sodium lauryl sulfate.

Glidants are used to promote powder flow by reducing interparticle friction and cohesion. These are used in combination with lubricants as they have no ability to reduce die wall friction. Glidants may be, for example, colloidal silicon dioxide, calcium silicate, calcium phosphate tribasic.

A very important parameter for the pharmaceutical industry is the achievement of linearity between the strength and the formulation. The present invention provides solid dosage forms that exhibit linearity between the strength of the drug formulation and the total mass of the formulation, by proportional increase of the amounts of the drug substance and the excipients in the formulation.

Another embodiment of the present invention is the use of a cost effective process for the preparation of solid dosage forms such as tablets containing Darunavir propylene glycolate.

Thus, according to the present invention, the use of a wet granulation process for the preparation of solid dosage forms such as tablets containing Darunavir propylene glycolate was used since standard equipment are involved and the tablets produced by this method are also characterized by satisfactory physical resistance with no need for special packaging.

The tablets of the present invention can also have a film coating applied on the core that improves appearance as well as swallowability of the dosage form.

### EXAMPLES

### Example 1 (not claimed)

Microcrystalline cellulose was used in the formulation of example 1 as a diluent in order to provide compressibility and improve flow properties of the powder blend. Both direct compression and wet granulation were applied as manufacturing processes and the formulations were tested for their physical characteristics.

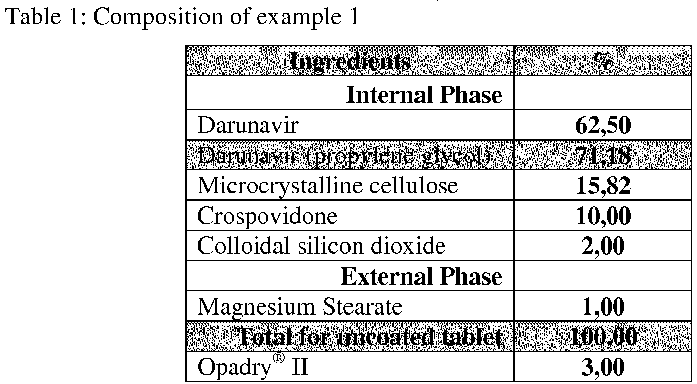

### Manufacturing process 1A (direct compression):

1. Mixing the API with MCC, Crospovidone and Colloidal silicon dioxide.
2. Addition of Magnesium Stearate and mixing for 3 min.
3. Compression to tablets
4. Film-Coating of tablets

### Manufacturing process 1B (wet granulation):

1. Mixing the API with MCC, Crospovidone and Colloidal silicon dioxide.
2. Kneading with water and then drying at 40°C.
3. Addition of Magnesium Stearate and mixing for 3 min.
4. Compression to tablets
5. Film-Coating of tablets

**Table 2: Physical characteristics of tablets of example 1**

| **RESULTS** | | |
|---|---|---|
| | **Trial 1A** | **Trial 1B** |
| **Hardness** | 175N | 293N |
| **Disintegration** | 0'17" | 2'08" |
| **Carr's index** | 29% | 17% |

### Example 2 (not claimed)

For tablet of example 2, microcrystalline cellulose was substituted with lactose spray dried in order to lower hardness and disintegration time. Both dry mixing (2A) and wet granulation (2B) processes as above were applied and physical properties of the tablets were tested.

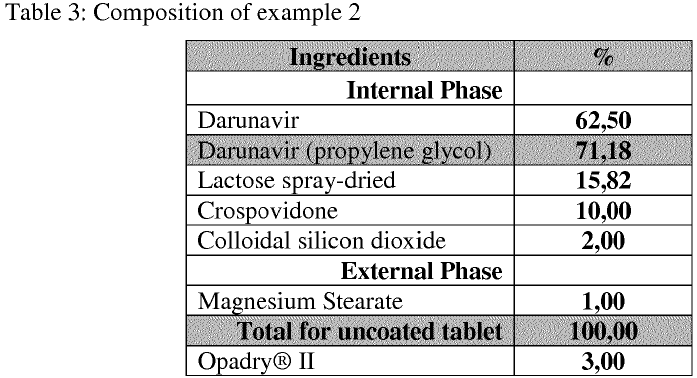

**Table 4: Physical characteristics of tablets of example 2**

| **RESULTS** | | |
|---|---|---|
| | **Trial 2A** | **Trial 2B** |
| **Hardness** | 101N | 173N |
| **Disintegration** | 0'12" | 0'90" |
| **Carr's index** | 28% | 22% |

### Example 3 (not claimed)

A physical mixture of microcrystalline cellulose and lactose in a ratio 1 to 3 was studied for tablets of example 3 in order to combine the beneficial properties of the two excipients as presented in previous examples. Again both dry mixing (3A) and wet granulation (3B) processes as above were applied and physical properties of the tablets were tested.

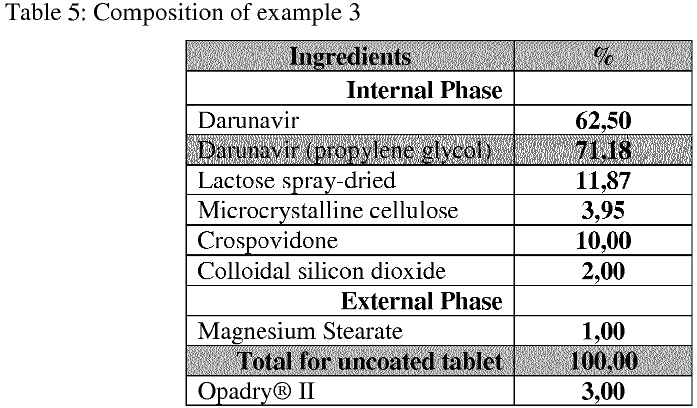

**Table 6: Physical characteristics of tablets of example 3**

| **RESULTS** | | |
|---|---|---|
| | **Trial 3A** | **Trial 3B** |
| **Hardness** | 115N | 191N |
| **Disintegration** | 0'17" | 1'14" |
| **Carr's index** | 27% | 19% |

### Example 4 (not claimed)

The co-spray dried mixture of microcrystalline cellulose with lactose sold under the tradename Microcelac® 100 was subsequently tested as diluent. As in previous example two manufacturing processes were tested and the physical properties of the tablets were measured.

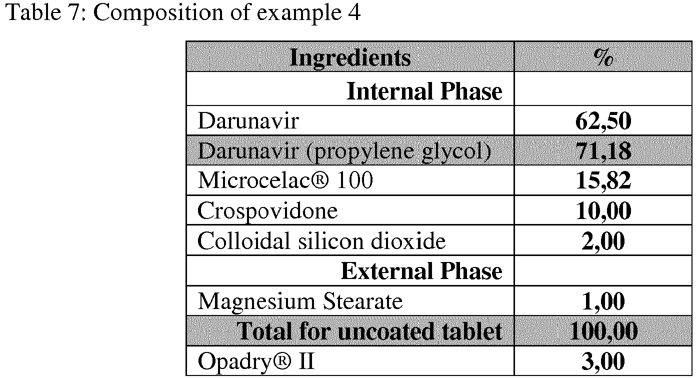

**Table 8: Physical characteristics of tablets of example 4**

| **RESULTS** | | |
|---|---|---|
| | **Trial 4A** | **Trial 4B** |
| **Hardness** | 167N | 201N |
| **Disintegration** | 0'34" | 1'14" |
| **Carr's index** | 33,3% | 16% |

### Example 5

As it is obvious from the examples above that wet granulation process provides the most desirable physical characteristics of the tablets produced. However, in order to further increase the hardness and improve the compaction properties of the blend Povidone was used as binder in amounts of 1%, 2%, 5% & 7%. Also the amount of crospovidone was reduced to 8% and 6% since disintegration was very fast.

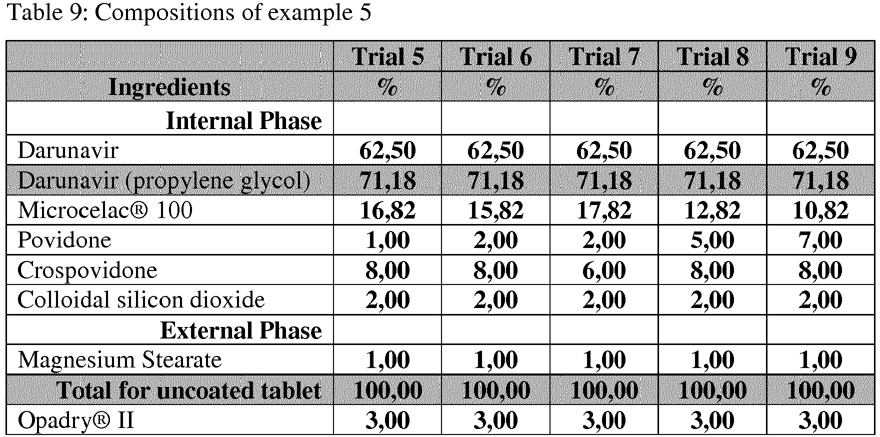

### Manufacturing process

1. Mixing the API with Microcelac® 100, Crospovidone, and Colloidal silicon dioxide.
2. Dissolving Povidone in water
3. Kneading with Povidone solution and then drying at 40°C.
4. Addition of Magnesium Stearate and mixing for 3 min.
5. Compression and film-Coating of tablets

**Table 10: Physical characteristics of tablets of example 5**

| **RESULTS** | | | | | |
|---|---|---|---|---|---|
| | **Trial 5** | **Trial 6** | **Trial 7** | **Trial 8** | **Trial 9** |
| **Hardness** | 229N | 219N | 221N | 235N | 280N |
| **Disintegration** | 1'38" | 1'41" | 1'40" | 2'41" | 4'30" |
| **Carr's index** | 17% | 17% | 16,7% | 17% | 19% |

The tablets of example 5 were tested also for their dissolution profile in a paddles apparatus, having 900ml of buffer with pH 3 and 2% Tween® 20 at 75rpm speed. The results are presented in table 11 below.

**Table 11: Dissolution profile of tablets of example 5**

| | **800mg** | | | | |
|---|---|---|---|---|---|
| **Time (min)** | **Trial 5 % release** | **Trial 6 % release** | **Trial 7 % release** | **Trial 8 % release** | **Trial 9 % release** |
| **5** | 48,89 | 56,73 | 53,76 | 44,02 | 38,98 |
| **10** | 73,87 | 74,04 | 75,43 | 65,57 | 55,95 |
| **15** | 79,81 | 82,60 | 85,67 | 77,88 | 66,45 |
| **20** | 84,77 | 89,54 | 91,16 | 84,69 | 70,45 |
| **30** | 91,83 | 93,52 | 95,63 | 92,85 | 80,20 |
| **45** | 99,27 | 98,76 | 98,14 | 96,55 | 86,48 |

It can be concluded that the use of Povidone in amounts up to 5% provide acceptable physical characteristics. The tablets of trial 7 presented the most favorable dissolution profile out of the three composition of the present example. Thus, they were stored under normal, intermediate and accelerated conditions and were examined for impurities in appropriate time points in order to test their stability.

**Table 12: Stability results of tablets of trial 7**

| | **Zero time** | **6 MONTHS** | | |
|---|---|---|---|---|
| **SPECIFICATION** | | **25° C/60% RH** | **30° C/65% RH** | **40° C/75% RH** |
| **Total impurities** | 0.07% | 0.16% | 0.22% | 0.34% |

The results presented above confirm that tablets of trial 7 are stable after 6 months storage under normal, intermediate and accelerated conditions.

While the present invention has been described with respect to the particular embodiments, it will be apparent to those skilled in the art that various changes and modifications may be made in the invention without departing from the spirit and scope thereof, as defined in the appended claims.

## Claims

1. Darunavir propylene glycolate immediate release tablet comprising an effective amount of a co-spray-dried mixture of lactose and microcrystalline cellulose and povidone in an amount of from 1% to 5% by weight of the total weight of the composition.

2. The immediate release tablet of claim 1, wherein the co-spray-dried mixture consists of 75% by weight lactose and 25% by weight microcrystalline cellulose.

3. The immediate release tablet of claim 1, wherein the co-spray-dried mixture is an amount of from 10% to 20% by weight of the total weight of the composition.

4. The immediate release tablet of claim 1, wherein it further comprises a disintegrant in an amount of from 5% to 15% by weight of the total weight of the composition.

5. The immediate release tablet of claims 3 and 4, wherein it further comprises a glidant and a lubricant.

6. The immediate release tablet of claim 5, wherein the disintegrant is Crospovidone, the glidant is colloidal silicon dioxide and lubricant is magnesium stearate.

7. A process for the preparation of an immediate release tablet Darunavir propylene glycolate as the active ingredient and an effective amount of a co-spray-dried mixture of lactose and microcrystalline cellulose and povidone in an amount of from 1% to 5% by weight of the total weight of the composition, wherein said process comprises the steps of:
- Mixing the Darunavir propylene glycolate with the co-spray-dried mixture of lactose and microcrystalline cellulose, a glidants and a disintegrant;
- Dissolving povidone in water;
- Kneading with the povidone solution the mixture of the first stage and then drying at 40°C;
- Addition of a lubricant and mixing for 3 min;
- Compression of the granule into tablets and optionally applying a film-coating.

8. The process according to claim 7, wherein the co-spray-dried mixture consists of 75% by weight lactose and 25% by weight microcrystalline cellulose.

9. The process according to claim 7, wherein the co-spray-dried mixture is an amount of from 10% to 20% by weight of the total weight of the composition.

## Patentansprüche

1. Darunavir-Propylenglycolat-Tablette mit sofortiger Freisetzung bestehend aus einer effektiven Menge einer co-sprühgetrockneten Mischung von Lactose und mikrokristalliner Cellulose und Povidon in einem Gewichtsprozent zwischen 1% und 5% des gesamten Gewichts der Zusammensetzung.

2. Tablette mit sofortiger Wirkstofffreisetzung nach Anspruch 1, wobei die co-sprühgetrocknete Mischung aus einem Gewichtsprozent von 75% aus Lactose und 25% aus mikrokrystalliner Cellulose besteht.

3. Tablette mit sofortiger Wirkstofffreisetzung nach Anspruch 1, wobei die co-sprühgetrocknete Mischung einen Gewichtsprozent von 10% bis 20% des gesamten Gewichts der Zusammensetzung umfasst.

4. Tablette mit sofortiger Wirkstofffreisetzung nach Anspruch 1, die weiterhin ein Spreng-/Zerfallsmittel in einem Gewichtsprozent von 5% bis 15% des gesamten Gewichts der Zusammensetzung umfasst.

5. Tablette mit sofortiger Wirkstofffreisetzung nach Ansprüchen 3 und 4, die weiterhin ein Gleitmittel und ein Schmiermittel umfasst.

6. Tablette mit sofortiger Wirkstofffreisetzung nach Anspruch 5, wobei das Spreng-/Zerfallsmittel Crospovidon, das Gleitmittel kolloidales Siliciumdioxid und das Schmiermittel Magnesiumstearat ist.

7. Verfahren für die Zubereitung einer Tablette mit sofortiger Wirkstofffreisetzung bestehend aus Darunavir-Propylenglycolat als Wirkstoff sowie aus einer effektiven Menge einer co-sprühgetrockneten Mischung von Lactose und mikrokristalliner Cellulose in einem Gewichtsprozent von 1% bis 5% des gesamten Gewichts der Zusammensetzung, wobei das o.g. Verfahren die nachstehenden Schritte umfasst:
- Darunavir-Propylenglycolat mit der co-sprühgetrockneten Mischung von Lactose und mikrokristalliner Cellulose, das Gleitmittel und das Spreng-/Zerfallsmittel mischen;
- Povidon in Wasser auflösen;
- Die Mischung vom ersten Schritt mit Povidon kneten und dann bei 40°C trocknen;
- Ein Schmiermittel hinzufügen und für 3 Minuten vermischen;
- Das Granulat durch Kompression tablettieren und optional die Tabletten mit Film überziehen.

8. Verfahren nach Anspruch 7, wobei die co-sprühgetrocknete Mischung aus einem Gewichtsprozent von 75% aus Lactose und 25% aus mikrokrystalliner Cellulose besteht.

9. Verfahren nach Anspruch 7, wobei die co-sprühgetrocknete Mischung einen Gewichtsprozent von 10% bis 20% des gesammten Gewichts der Zusammensetzung umfasst.

## Revendications

1. Comprimé de darunavir propylène glycol à libération immédiate comprenant une quantité efficace d'un mélange séché par atomisation de lactose, de cellulose microcristalline et de povidone en une quantité de 1% à 5% en poids du poids total de la composition.

2. Le comprimé à libération immédiate de la 1ère revendication où le mélange séché par atomisation consiste à 75% en poids de lactose et à 25% en poids de cellulose microcristalline.

3. Le comprimé à libération immédiate de la 1ère revendication, où le mélange séché par atomisation consiste en une quantité de 10% é 20% en poids du poids total de la composition.

4. Le comprimé à libération immédiate de la 1ère revendication, qui comprend, en plus, un désintégrant en une quantité de 5% à 15% en poids du poids total de la composition.

5. Le comprimé à libération immédiate des revendications 3 et 4, qui comprend, en plus, un glissant et un lubrifiant.

6. Le comprimé à libération immédiate de la 4ème revendication, dans lequel le désintégrant est la Crospovidone, le glissant est le dioxyde de silicium colloïdal et le lubrifiant est le stéarate de magnésium.

7. Un procédé pour la préparation d'un comprimé Darunavir propylène glycol en tant qu'ingrédient actif et une quantité efficace d'un mélange séché par atomisation de lactose, de cellulose microcristalline et de povidone en une quantité de 1% à 5% en poids du poids total de la composition, qui comprend les étapes suivantes:
- Combinaison du Darunavir propylène glycol avec le mélange séché par atomisation de lactose, et de cellulose microcristalline, un glissant et un désintégrant;
- Dissolution de la povidone dans l'eau;
- Pétrissage avec la solution de povidone du mélange de la première étape puis séchage à 40°C;
- Ajout d'un lubrifiant et mélange pendant 3 minutes;
- Fabrication des comprimés à partir des granules, et, facultativement, pelliculage.

8. Le procédé conformément à la 7ème revendication, où le mélange séché par atomisation consiste à 75% en poids de lactose et à 25% en poids de cellulose microcristalline.

9. Le procédé conformément à la 7ème revendication, où le mélange séché par atomisation consiste en une quantité de 10% é 20% en poids du poids total de la composition.
